# EUROPÄISCHE PATENTSCHRIFT

(11) **EP 1 393 712 B1**
(45) Veröffentlichungstag und Bekanntmachung des Hinweises auf die Patenterteilung: **06.12.2006**
(21) Anmeldenummer: 03015949.5
(22) Anmeldetag: 14.07.2003
(51) Int. Cl.: A61Q 15/00, A61K 8/26, A61K 8/28, A61K 8/81

(54) **Kosmetische und dermatologische Antitranspirantzubereitungen enthaltend einen tonmineralischen Verdicker und ein Polyolefin**
Cosmetic and dermatological antiperspirant compositions containing a clay thickening system and a polyolefine
Compositions antisudorifiques cosmétiques et dermatologiques contenant un épaississant argileux et une polyoléfine

(30) Priorität: 09.08.2002 DE 10237054
(43) Veröffentlichungstag der Anmeldung: 03.03.2004
(73) Patentinhaber: Beiersdorf AG, 20245 Hamburg (DE)
(72) Erfinder: Schulz, Ulrike, 20253 Hamburg (DE); Kux, Ulrich, Dr., 22559 Hamburg (DE)

(56) Entgegenhaltungen:
- WO-A-97/16161
- US-A- 5 976 514
- US-B1- 6 428 777

## Beschreibung

Die vorliegende Erfindung betrifft eine kosmetische und dermatologische AntitranspirantZubereitung mit tonmineralhaltigem Verdickersystem.

Die menschliche Haut ist mit zwei bis drei Millionen Schweißdrüsen durchsetzt. Diese sind Tag und Nacht im Einsatz, um Feuchtigkeit an die Hautoberfläche zu befördern und so der Überhitzung des Organismus vorzubeugen. Der austretende Schweiß sorgt bei seiner Verdunstung für die notwendige Kühlung.

Täglich werden im Normalfall 0,5 bis 1 Liter Schweiß produziert. Bei Belastung des Körpers und erhöhtem Stoffwechsel kann es ein Vielfaches an produziertem Schweiß sein. Im Laufe der Entwicklung eines Menschen kommt es zur Ausbildung zweier Arten von Schweißdrüsen. Von Geburt an besitzt der Mensch nur ekkrine Schweißdrüsen (kleine Schweißdrüsen), mit Beginn der Pubertät jedoch kommt es hauptsächlich im Bereich der Achseln und im Anal- und Genitalbereich zur Ausbildung der apokrinen Schweißdrüsen (große Schweißdrüsen). Erst diese sorgen in Zusammenhang mit Hautbakterien, die den geruchlosen Schweiß zersetzen, zu den bekannten unangenehmen Gerüchen. Der Schweißgeruch ist personenspezifisch und bei jedem Menschen unterschiedlich ausgeprägt. Durch einfaches Waschen lässt sich bei den meisten Menschen nur eine kurzfristige Besserung erzielen, so dass es oft genug nicht ohne die Anwendung von Deowirkstoffen geht.

Um eine Deowirkung zu erreichen gibt es verschiedene Wege, die im Normalfall kombiniert angewendet werden.

Der Einsatz von Antitranspirantien, die die Schweißproduktion durch Blockierung der Schweißdrüsenausgänge verhindern, ist schon lange bekannt. Verwendung finden hier in aller Regel Aluminium- und Aluminium/Zirkoniumsalze. Die verringerte Schweißproduktion hat nach neuesten Erkenntnissen keine Auswirkung auf den Organismus, da die ,Kühlwirkung' zum großen Teil über das ,Schwitzen' der anderen Hautpartien (ekkrine Drüsen) erfolgt.

Die Hemmung des Bakterienwachstums durch Bakteriostatika im Bereich der mit apokrinen Schweißdrüsen durchsetzten Hautzonen ist nicht unbedenklich und führt zum Teil zu starken Reizungen und allergischen Reaktionen.

Als Bakteriozid wirkt auch der Alkohol (Ethanol), der in vielen der herkömmlichen Deoprodukte enthalten ist. Auch hier sind Nebenwirkungen häufig.

Zur Maskierung des Schweißgeruchs sind in der Regel Duftstoffe bzw. Parfümstoffe in den Deozubereitungen vorhanden. Diese wirken zum Teil auch bakteriostatisch, haben aber bei vielen Anwendern ähnliche Nebenwirkungen wie die Bakteriostatika.

Aufgrund der zuvor beschriebenen Wirkungsweise und den damit einhergehenden Nebenwirkungen sind die Bestrebungen groß, Deoprodukte zu entwickeln, die keine Nebenwirkungen aufweisen. Der Trend geht also eindeutig in Richtung alkohol- und wasserfreier Antitranspirantprodukte, bei denen eine antitranspirante Wirkung mit hautpflegender Wirkung einhergeht. Diese weisen eine zu Emulsionen deutlich unterschiedliche Sensorik auf. Gleichzeitig sollen die Zubereitungen eine Konsistenz besitzen, die eine Auftragung durch gebräuchliche Deoroller ermöglicht.

Die Kombination der zuvor genannten Merkmale mit dem Wunsch nach gut wirksamen Antitranspirantprodukten ließ sich bislang z.B. mit wässrig-alkoholischen Rezepturen realisieren. Diese Formulierungen bestehen praktisch nur aus Wasser und Alkohol als Medium, Deo- und Antitranspirantmittel als Wirkstoffe sowie Parfüm, Löslichkeitsvermittler und Verdicker (zumeist auf Kohlenhydratbasis) als zusätzliche Agenzien. Sie werden vom Verbraucher als frisch und kühlend empfunden, sind aber gleichzeitig mit einer ganzen Reihe an Mankos behaftet. So ist die Applikation vor allem auf frisch rasierter Haut durch den Alkoholgehalt mit Unverträglichkeiten verbunden. Ein weiterer großer Nachteil ist die Tatsache, dass in derartige Systeme keine größeren Ölmengen eingearbeitet werden können. Durch den für eine hocheffektive Wirkleistung erforderlichen hohen Gehalt an Antitranspirantsalz verbleibt nach der Applikation auf der Haut ein weißer Rückstand, der vom Verbraucher als überaus störend empfunden wird. Durch die technologisch bedingte Abwesenheit einer ausreichendend großen Ölphase kann dieser allerdings nicht kaschiert werden. Darüber hinaus führt die Verwendung von Kohlenhydrat-Verdickern zu einer gewissen Klebrigkeit des Produktes nach dem Verdunsten des Alkohols. Zusammenfassend kann man sagen, dass wässrig-alkoholische Rezepturen nicht als Grundlage für die Einarbeitung hoher Gehalte an Antitranspirantwirkern (Aluminium- bzw. Aluminium/Zirkonium-Komplexe) geeignet sind.

Eine weitere wichtige markteingeführte Technologie im Bereich der Antitranspirantprodukte basiert auf Emulsionen. Die weisen gegenüber den wässrig-alkoholischen Rezepturen bereits deutliche Vorteile auf. So kann mit Hilfe von Emulgatoren eine Ölphase stabil eingearbeitet und diese darüber hinaus so gewählt werden, daß sowohl eine zufriedenstellende Kaschierung des weißen Rückstands als auch eine Minimierung der Klebrigkeit gelingt. Diese Produkteigenschaften sind durch Einarbeitung des wasserlöslichen Antitranspirantwirkers bedingt und werden beide vom Verbraucher als unangenehm und störend empfunden, falls es formulierungstechnisch nicht gelingt, diesbezüglich erfolgreiche Gegenmaßnahmen zu treffen. Zu diesem Zwecke werden gerne Mischungen aus nicht-flüchtigen (zur Kaschierung) und flüchtigen Ölen (für ansprechende Sensorik) eingesetzt.

Ein weiterer großer Vorteil von Emulsionen gebenüber wässrig-alkoholischen Zubereitungen ist eine deutliche verbesserte Hautverträglichkeit, die durch das Fehlen von Alkohol und dem okklusiven Effekt der eingearbeiteten Öle bewerkstelligt wird.

Es verbleibt allerdings eine generische Schwäche der emulsionsbasierenden Produkte, die viele Konsumenten davon abhält, diese an sich vorteilhaften und auch ökologisch unbedenklichen Zubereitungen zu favorisieren. Der hohe Wasseranteil bedingt ein -gegenüber wasserfrei formulierten Produkten - scheinbar langsameres Einzugsvermögen, was in einem feuchten Hautgefühl resultiert. Dieses ist für viele Verbraucher nicht akzeptabel.

Viele Verbraucher favorisieren für die Auftragung von Antitranspirantien die Produktform der sogenannten Roll-ons, da sich mit ihnen das Füllgut fein verteilt in die Achselregion aufbringen lässt, ohne dass die Finger mit diesem in Berührung gebracht werden müssen. Gegenüber Aerosolen besteht weiterhin der ökologische Vorteil, dass Roll-ons ohne die Verwendung von Treibmitteln (verflüssigte Gase) auskommen.

Betrachtet man hierbei die Untergruppe der wasserfreien Roll-ons, so gilt es zu beachten, dass eine Flüssigkeit niedriger Viskosität ein leichteres Absetzen der suspendierten Feststoffteilchen infolge der Schwerkraft bedingt. Die abgesetzten Teilchen können durch eine feste Kuchenpackung schwer wieder aufschüttelbar sein.

Procter & Gamble beanspruchen mit EP0485012 die Bereitstellung von Produkten mit verbesserter Hautverträglichkeit. Realisiert werden soll dies durch Zubereitungen beinhaltend eine Matrixphase aus hohen Mengen an nichtflüchtigen Silikonen in Kombination mit niedrigen Mengen an flüchtigen Silikonen; über die Art der Verdickung dieser Matrixphase werden jedoch keine speziellen Angaben gemacht. Zubereitungen dieser Art führen durch die hohen Anteile nichtflüchtiger Öle zu einem langsamen Einzugsvermögen und einem öligen, fettigen Hautgefühl.

Die EP0028853 von Procter & Gamble beansprucht eine ähnlich aufgebaute Matrixphase.

Procter & Gamble beanspruchen mit WO0030598 ebenfalls Produkte mit verbesserter Verträglichkeit. In diesem Falle besteht die Matrixphase bevorzugt aus einem Gemisch aus flüchtigen unpolaren Kohlenwasserstoffen und nichtflüchtigen Silikonen wie Dimethicone. Diese Zubereitungen sind jedoch für die Anwendung in festen Stiften, halbfesten Cremes und Aerosolen vorgesehen und vermitteln ebenfalls ein öliges, fettiges Hautgefühl und ziehen langsam ein.

Unilever stellte mit EP0319168 Zubereitungen, enthaltend flüchtige Silikone als Fluid und Bentone SD-1 Tonmineral als Dispergierhilfe vor, die eine sehr hohe Antitranspirant-Leistung versprechen; jedoch ist das Absetzverhalten des zur Verdickung verwendeten Bentone-Systems mangelhaft.

US-B1-6 428 777 offenbart eine Antitranspirantzubereitung, enthaltend ein Verdickersystem, enthaltend (Spalte 7, Zeile 20-24; Beispiele C-D):
- einen oder mehrere tonmineralische Verdicker A (Bentone, Hectorite...),
- ein oder mehrere Polyolefine als Verdicken B (Polydecen),
- einen oder mehrere nicht-wässrige polare Aktivatoren C (Ethanol, Propylencarbonat, Fragrance) und
- Wasser,
einen oder mehrere Antitranspirantwirkstoffe (AlZr) und
eine Matrixphase,
wobei die Matrixphase mindestens ein Silikonöl enthält (Cyclomethicone) und das Gewichtsverhältnis von tonmineralischen Verdickern A zu nicht-wässrigen polaren Aktivatoren C aus dem Bereich von 3 : 1 bis 5 : 1 gewählt wird.

Die markteingeführten Produkte weisen meist vom Verbraucher als störend empfundene Merkmale auf:
- schnelle Phasentrennung,
- starke äußere Verölung des Packmittels und
- Kuchenbildung

Aufgabe der vorliegenden Erfindung ist es daher, den Stand der Technik zu bereichern, seinen Nachteilen abzuhelfen und dem Verbraucher Antitranspirant-Roller mit Zubereitungen niederer Viskosität, basierend auf einem tonmineralhaltigen Verdickersystem, zur Verfügung zu stellen.

Erfindungsgemäße Zubereitungen zeichnen sich durch eine einzigartige Sensorik aus. Sie fühlen sich samtig und weich an, kleben nicht, trocknen schnell und erzeugen ein nichtkühlendes, wohliges Hautgefühl. Der Frischeeindruck, der bei Deos ja nicht völlig verloren gehen darf, muß in diesem Falle über das Parfüm eingebracht werden. Sie sind ein Angebot an Verbraucher, die das feuchte Hautgefühl der üblichen wasserhaltigen Roller nicht mögen und deshalb zu Aerosolen, Antitranspirantstiften oder sog. Soft Solids (wasserfreie Cremes) greifen, aber prinzipiell auch an Rollern interessiert sind oder diese - nach Behebung der Mängel des Stands der Technik - sogar bevorzugen würden.

Es hat sich gezeigt, dass die Viskosität der flüssigen Matrixphase mit Hilfe eines Verdickersystems, enthaltend
- einen oder mehrere tonmineralische Verdicker A,
- ein oder mehrere Polyolefine als Verdicker B,
- einen oder mehrere nicht-wässrige polare Aktivatoren C und
- Wasser,
und einen oder mehrere Antitranspirantwirkstoffe, wobei die Matrixphase mindestens ein Silikonöl enthält und das Gewichtsverhältnis von tonmineralischen Verdickern A zu nicht-wässrigen polaren Aktivatoren C aus dem Bereich von 3 : 1 bis 5 : 1, bevorzugt 4 : 1 gewählt wird, eine hervorragende Grundlage für Antitranspirant Zubereitungen bildet.

In Zubereitungen dieser Zusammensetzung ist die Absetzgeschwindigkeit der Feststoffe so eingestellt, daß die vom Verbraucher als störend empfundene Trennung des Produktes in verschiedene Phasen gegenüber markteingeführten Suspensions-Rollern signifikant verlangsamt wird.

In handelsüblichen Suspensions-Rollern wird die Viskositätserhöhung der Matrixphase ausschließlich durch tonmineralische Verdickersysteme erzielt. Durch die bei Produktanwendung auftretenden Scherkräfte verlieren solche tonmineralischen Verdickersysteme kurzfristig reversibel ihren stabilisierenden Effekt (Thixotropie), d.h. die Viskosität sinkt deutlich. Dies ist gewünscht, denn hierdurch wird der Produktaustrag erleichtert. Die Schwäche solcher Systeme liegt nun aber darin, daß bereits durch ungewolltes Schütteln des Produktes, wie es z.B. in einer Sporttasche erfolgt, die Viskosität ebenfalls erniedrigt wird. Befindet sich das Produkt zudem im liegenden Zustand, kommt es bei marktüblichen Deorollerbehältnissen hierdurch unvermeidbar zum ungewollten Produktaustrag, was eine vom Verbraucher als äußerst unangenehm empfundene äußere Verölung des Packmittels nach sich zieht. Die Folge davon ist, dass handelsübliche Suspensions-Roller nicht gut für eine flexible Nutzung auch außerhalb der üblichen häuslichen Anwendung geeignet sind.

Hier schafft nun das erfindungsgemäße Verdickersystem, bestehend aus einem scherempfindlichen tonmineralischen Verdicker A in Kombination mit einem scherunempfindlichen Verdicker B, Abhilfe.

Durch Verwendung des zusätzlichen Verdickers B bleibt nun überraschenderweise und nicht vorhersehbar die Viskosität - und somit die Stabilisierung der Füllgutfließgrenze - auch bei ungewollter Scherung hoch genug. Die Folge davon ist, dass das äußere Verölen des Packmittels gänzlich ausbleibt oder packmittelabhängig zumindest deutlich reduziert wird. Zusätzlich wird - wie bereits erwähnt - die Absetzgeschwindigkeit der suspendierten Feststoffe und somit die Trennung des Füllgutes in 2 Phasen signifikant gegenüber handelsüblichen Suspensions-Rollern verlangsamt.

Als Verdicker A können modifizierte Schichtsilikate und/oder Tonmineralien eingesetzt werden.

Silikate sind Salze und Ester (Kieselsäureester) der Orthokieselsäure [Si(OH)₄] und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten. Schichtsilikate (Phyllosilikate, Blattsilikate) sind (idealerweise) Silikat-Strukturen mit zweidimensional unendlichen Schichten aus [SiO₄]⁴⁻-Tetraedern,wobei jedes Tetraeder über 3 Brücken-Sauerstoffe mit Nachbar-Tetraedern verbunden ist.

Chemische Formeln lassen sich für Schichtsilikate nur angenähert aufstellen, da sie ein großes lonenaustausch-Vermögen besitzen und Silizium gegen Aluminium und dieses wiederum gegen Magnesium, Fe²⁺, Fe³⁺, Zn und dergleichen ausgetauscht werden kann. Die daraus möglicherweise resultierende negative Ladung der Schichten wird in der Regel durch Kationen, insbesondere durch Na⁺ und Ca²⁺ in Zwischenschicht-Positionen ausgeglichen.

Schichtsilikate können durch reversible Einlagerung von Wasser (in der 2- bis 7-fachen Menge) und anderen Substanzen wie z. B. Alkoholen, Glykolen und dergleichen mehr aufquellen. Ihre Verwendung als Verdickungsmittel in kosmetischen Mitteln ist dementsprechend an sich bekannt. Allerdings konnte der Stand der Technik nicht den Weg zur vorliegenden Erfindung weisen.

Vorteilhafte Schichtsilikate sind beispielsweise solche, deren größte Ausdehnungsrichtung im unmodifizierten und ungequollenen Zustand im Mittel eine Länge von weniger als 10 µm hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Substanzen ab.

Vorteilhafte modifizierte Schichtsilikate im Sinne der vorliegenden Erfindung sind beispielsweise modifizierte Smektite (Smectite).

Smektite sind stets sehr feinkörnige (meist < 2 mm), überwiegend als lamellenförmige, moosartige oder kugelförmige Aggregate vorkommende Dreischicht-Tonminerale (2:1-Schichtsilikate), in denen eine zentrale Schicht aus oktaedrisch koordinierten Kationen sandwichartig von 2 Schichten aus [(Si,Al)O₄]-Tetraedern umgeben ist. Smektite werden idealisiert durch die folgende Strukturformel beschrieben, worin weiß ausgefüllte Kreise Silizium- und/oder Aluminiumatome, hellgrau ausgefüllte Kreise Sauerstoffatome, dunkelgrau ausgefüllte Kreise Wasserstoffatome und schwarz ausgefüllte Kreise Aluminium-, Magnesium-, Eisenatome und/oder weitere Austauschkationen darstellen:

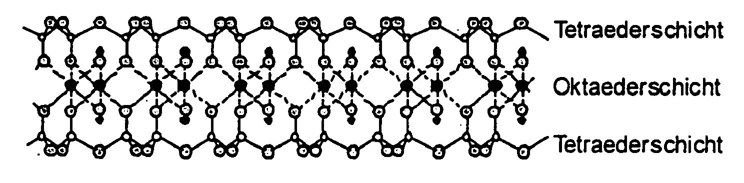

Vorteilhafte modifizierte Smektite sind z. B. modifizierte Montmorillonite. Montmorillonite werden durch die angenäherte chemische Formel Al₂[(OH)₂/Si₄O₁₀] · n H₂O bzw. Al₂O₃ · 4 SiO₂ · H₂O n H₂O beschrieben und stellen zu den dioktaedrischen Smektiten gehörende Tonmineralien dar.

Besonders vorteilhaft im Sinne der vorliegenden Erfindung sind ferner beispielsweise modifizierte Hektorite. Hektorite gehören zu den Smektiten und haben die angenäherte chemische Formel M⁺_{0.3}(Mg_{2.7}Li_{0.3})[Si₄O₁₀(OH)₂], worin M⁺ meist Na⁺ darstellt.

Vorteilhaft im Sinne der vorliegenden Erfindung sind ferner modifizierte Bentonite. Bentonite sind Tone und Gesteine, die Smektite, vor allem Montmorillonit, als Hauptminerale enthalten. Die "Roh"-Bentonite sind entweder Calcium-Bentonite (in Großbritannien als Fuller-Erden bezeichnet) oder Natrium-Bentonite (auch: Wyoming-Bentonite).

Modifizierte Schichtsilikate im Sinne der vorliegenden Erfindung sind Schichtsilikate, insbesondere die bereits genannten Schichtsilikattypen, deren Organophilie (auch: Lipophilie)-beispielsweise durch Umsetzung mit quarternären Ammonium-Verbindungen - erhöht wurde. Solche Schichtsilikate werden auch als organophile Schichtsilikate bezeichnet.

Besonders vorteilhaft im Sinne der vorliegenden Erfindung sind sogenannte Bentone, d. h. organische Derivate von Montmorilloniten (bzw. Bentoniten) und/oder Hektoriten, die durch lonenaustausch-Reaktionen mit Alkylammonium-Basen hergestellt werden.

Vorteilhafte modifizierte Schichtsilikate im Sinne der vorliegenden Erfindung sind beispielsweise durch Umsetzung von Schichtsilikaten mit Quaternium-18 erhältlich. Quaternium-18 ist eine Mischung von quaternären Ammoniumchloridsalzen, welche durch die folgende Strukturformel beschrieben werden: 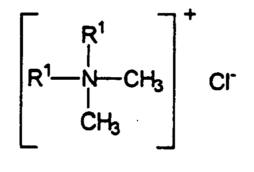 worin die Reste R¹ unabhängig voneinander gewählt werden aus hydrierten Talgresten mit einer Kettenlänge von 12 bis 20 Kohlenstoffatomen.

Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18, welche z. B. unter den Handelsbezeichnungen Bentone 27 und Bentone 38 bei Nordmann & Rassmann erhältlich sind.

Ganz besonders bevorzugt ist erfindungsgemäß Quaternium-90 Bentonite, ein Reaktionsprodukt aus Bentonite und Quaternium-90, das von der Firma Süd-Chemie unter dem Handelsnamen Tixogel VP-V zu beziehen ist. Die Bezeichnung deutet daraufhin, daß die Alkylreste R¹ bei diesem Produkt pflanzlichen Ursprungs sind, wodurch sich besonders vorteilhafte Eigenschaften im Sinne der vorliegenden Erfindung bzgl. Verdickung der Matrixphase und Wiederaufschüttelbarkeit des suspendierten Antitranspirant-Wirkstoffes ergeben.

So ist die Kettenlängenverteilung der Alkylreste R¹ typisch für Rohstoffquellen wie hydriertes Palmöl. Der Anteil der Alkylreste R¹ ist wie folgt, wobei sich rohstoffbedingte Schwankungen aufgrund der natürlichen Herkunft ergeben:
C₁₈H₃₇ ca. 55-65 Gew.-%, C₁₆H₃₃ ca. 30-40 Gew.-%, C₁₄H₂₉ < 5 Gew.-%

Schichtsilikate und Tonmineralien wirken auf der Haut auch als Geruchsabsorber.

Die Gesamtmenge an einem oder mehreren modifizierten Schichtsilikaten und Tonmineralien in den fertigen Antitranspirant-Zubereitungen wird vorteilhaft aus dem Bereich von 0,05 bis 5,0 Gew.-%, bevorzugt von 0,5 bis 3,5 Gew.-%, besonders bevorzugt von 1,0 bis 3,0 Gew.-% gewählt, bezogen auf das Gesamtgewicht der Zubereitungen.

Durch die Kombination von kurzkettigen Polyolefinen aus der Gruppe Polyisobuten und dessen Copolymeren als Verdicker B mit tonmineralischen Verdickern A, verringert sich die Einsatzkonzentration an Tonmineral, da bereits die Zugabe geringer Mengen sehr effektiv die Viskosität der Matrixphase erhöht. Dadurch verringern sich zudem die üblicherweise mit tonmineralischen Verdickern verbundenen Nachteile wie Verfärbung und Geruch - bedingt durch unvermeidbare Spuren von Verunreinigungen in den Tonmineralien - der Zubereitung und die Formelkosten entsprechend proportional.

Handelsübliche Polyisobutene lassen sich in 3 Produktkategorien einordnen:
- ölige Flüssigkeiten (Molmasse: 300-3000 g/mol)
- zähflüssige, klebrige Massen (Molmasse: 40000-120000 g/mol)
- Kautschuk-artige, elastische Massen (Molmasse: 300000-2500000 g/mol).

Insbesondere Polyisobuten mit einem niederen Molekulargewicht von durchschnittlich 300 und 3000 g/mol entsprechend einer Monomerenzahl von 5 bis ca. 55 Einheiten ist hervorragend geeignet. Ebenso ist die Verwendung von Copolymeren aus Isobuten und anderen verzweigten und/oder unverzweigten Alkenen, die eine Kettenlänge von zwei bis 21 Kohlenstoffatomen aufweisen, erfindungsgemäß.

Erfindungsgemäße Polyisobutene sind unter anderem unter folgenden Handelsbezeichnungen erhältlich: REWOPAL PIB 1000 (Goldschmidt Rewo GmbH & Co.KG), Permethyl 104A (Presperse Inc.)

Die Gesamtmenge an Verdicker B in den fertigen Antitranspirant Zubereitung wird vorteilhaft aus dem Bereich von 0,05 bis 2,0 Gew.-%, bevorzugt von 0,5 bis 1,5 Gew.-%, besonders bevorzugt wie 1,0 Gew.-% gewählt, bezogen auf das Gesamtgewicht der Zubereitungen.

Ein wichtiger Baustein der erfindungsgemäßen Zubereitungen ist der sogenannte Aktivator. Diesem kommt die Aufgabe zu, das eingesetzte hydrophobierte Tonmineral zu delaminieren, was auch als Aktivierung bezeichnet wird. Üblicherweise werden hierzu kleine, polare Moleküle wie Propylenglycolcarbonat und Ethanol eingesetzt, die sich unter mechanischem Energieeintrag zwischen die Schichten der Tonminerallamellen schieben und somit den gewünschten Vorgang durch elektrostatische Wechselwirkung mit diesen ermöglichen. Darüber hinaus bilden sie Wasserstoffbrückenbindungen zu den delamellierten Tonmineralplättchen aus und sorgen durch diese Brückenfunktion - in einer Doppelfunktion als quasi Klammer und Scharnier - für den Zusammenhalt der entstehenden spielkartenhausähnlichen Struktur.

Die beschriebenen Vorgänge auf mikroskopischer Ebene bewirken eine Verdickung der flüssigen Matrix und spiegeln sich in einer makroskopisch leicht beobachtbaren Viskositätserhöhung des Systems wieder. Typischerweise zeigen diese Systeme eine stark ausgeprägte Thixotropie.

Ein weiterer erfindungsgemäßer Teilaspekt besteht in einem besonderen Gewichtsverhältnis von Tonmineralverdicker A zu Aktivator C. Die Effizienz und rheologische Charakteristik der Viskositätserhöhung erfindungsgemäßiger silikonölhaltiger Matrixphasen durch tonmineralische Verdicker A ist in entscheidendem Maße von auf das jeweilige System optimierten Abmischungverhältnissen und Herstellbedingungen abhängig. So kann ein nicht-optimiertes Gewichtsverhältnis von A zu C zu ungünstigen rheologischen Eigenschaften führen. Hierbei kann die Menge an A im Gewichtsverhältnis zu C sowohl zu niedrig als auch zu hoch sein, was sich in beiden Fällen in einer unzureichenden Verdickungsleistung und somit schneller Phasentrennung des Füllguts niederschlägt.

Das für erfindungsgemäße Zubereitungen bevorzugte Gewichtsverhältnis von scherempfindlichem tonmineralischem Verdicker A zu Aktivator C liegt im Bereich von 3 : 1 bis 5 : 1, bevorzugt wie 4 : 1.

Die Menge an zugesetztem Wasser in den Zubereitungen beträgt vorzugsweise 0,01 bis 2,0 Gew.-%, bevorzugt 0,05 bis 1,75 Gew.-%, besonders bevorzugt 0,5 bis 1,5 Gew.-%, bezogen auf das Gesamtgewicht der Zubereitung.

Unter zugesetztem Wasser ist diejenige Menge an Wasser zu verstehen, die in reiner Form im Herstellungsprozess zugegeben wird. Dieses Wasser dient ebenfalls - wie der Aktivator C - der Aktivierung des eingesetzen Tonminerals und wird herstellungsbedingt durch dieses so fest gebunden bzw. absorbiert bzw. adsorbiert, daß es mit herkömmlichen analytischen Methoden nicht wiederfindbar ist. Somit sind sie für verbrauchertypische Anwendungssituationen im Sinne einer technologischen Zuordnung als wasserfrei zu bezeichnen.

Die Hauptfunktion des Wassers in diesem System ist jedoch die eines ausgezeichneten Wasserstoffbrückenbildners, mit den bereits beschriebenen Funktionen als Klammer und Scharnier zwischen den delamellierten Tonmineralplättchen.

Die erfindungsgemäße Verwendung eines gemischten Aktivator-/Stabilisatorsystems, bevorzugt bestehend aus Propylenglycolcarbonat (Aktivator C) und Wasser (Gelstabilisator/ Aktivator D), ergibt mehrere Vorteile, wobei das Wasser als Teilsubstitut des Propylenglycolcarbonats gesehen werden kann. Durch den Einsatz von Wasser können die Formelkosten gesenkt und der dem Propylenglycolcarbonat eigene synthetische Geruch kann minimiert werden, so dass erfindungsgemäße Zubereitungen prinzipiell auch unparfümiert formuliert werden können.

Wasser, welches darüber hinaus in den erfindungsgemäßen Rohstoffen standardmäßig enthalten ist, ist nicht als zugesetztes Wasser zu betrachten, da es unter normalen Anwendungsbedingungen der beanspruchten Zubereitungen vom Verbraucher ebenfalls nicht sensorisch oder auf sonst eine Weise wahrnehmbar ist.

Vorteilhaft lassen sich große Mengen saurer Aluminium- und/oder Aluminium/Zirkoniumsalze stabil in die Grundformulierung einarbeiten. Es können 5 bis 40 Gew.-%, insbesondere 10 bis 25 Gew.-% Aluminiumchlorhydrat und/oder Aluminium/Zirkoniumchlorhydrat stabil eingearbeitet werden. Hierbei beziehen sich die beschriebenen Konzentrationsbereiche auf die sog. Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Die nachfolgende Auflistung vorteilhaft einzusetzender Antitranspirant-Wirker soll in keinster Weise einschränkend sein:

Aluminium-Salze (der empirischen Summenformel [Al₂(OH)ₘClₙ], wobei m+n=6):
- Aluminium-Salze wie Aluminiumchlorid AlCl₃, Aluminiumsulfat Al₂(SO₄)₃
- Aluminiumchlorhydrat [Al₂(OH)₅Cl)] x H₂O
   Standard Al-Komplexe: Locron L (Clariant), Chlorhydrol (Reheis), ACH-303 (Summit), Aloxicoll L (Giulini).
   Aktivierte Al-Komplexe: Reach 501 (Reheis), AACH-324 (Summit)
- Aluminiumsesquichlorhydrat [Al₂(OH)_{4,5}Cl_{1,5}] x H₂O
   Standard Al-Komplexe: Aluminum Sesquichlorohydrate (Reheis), ACH-308 (Summit), Aloxicoll 31 L (Giulini)
   Aktivierte Al-Komplexe: Reach 301 (Reheis)
- Aluminiumdichlorhydrat [Al₂(OH)₄Cl₂] x H₂O

Aluminium-Zirkonium-Salze:
- Aluminium/Zirkonium Trichlorhydrex Glycin [Al₄Zr(OH)₁₃Cl₃] x H₂O x Gly
   Standard Al/Zr-Komplexe: Rezal 33GP (Reheis), AZG-7164 (Summit), Zirkonal P3G (Giulini) Aktivierte Al/Zr-Komplexe: Reach AZZ 902 (Reheis), AAZG-7160 (Summit), Zirkonal AP3G (Giulini)
- Aluminium/Zirkonium Tetrachlorhydrex Glycin [Al₄Zr(OH)₁₂Cl₄] x H₂O x Gly
   Standard Al/Zr-Komplexe: Rezal 36G (Reheis), AZG-368 (Summit), Zirkonal L435G (Giulini) Aktivierte Al/Zr-Komplexe: Reach AZP 855 (Reheis), AAZG-6313-15 (Summit), Zirkonal AP4G (Giulini)
- Aluminium/Zirkonium Pentachlorhydrex Glycin [Al₈Zr(OH)₂₃Cl₅] x H₂O x Gly
   Standard Al/Zr-Komplexe: Rezal 67 (Reheis), Zirkonal L540 (Giulini)
   Aktivierte Al/Zr-Komplexe: Reach AZN 885 (Reheis)
- Aluminium/Zirkonium Octachlorhydrex Glycin [Al₈Zr(OH)₂₀Cl₈] x H₂O x Gly

Ebenso von Vorteil können aber auch Glycin-freie Aluminium/Zirkonium-Salze sein.

Dabei soll die Verwendung der Antitranspirant-Wirker aus den Rohstoffklassen Aluminium- und Aluminium/Zirkonium-Salzen nicht auf die handelsüblichen zumeist wäßrigen Lösungen, wie z.B. Locron L (Clariant), beschränkt sein, sondern es kann auch von Vorteil sein, die ebenfalls handelsüblichen wasserfreien Pulver derselbigen Rohstoffe durch Einbringung in die beanspruchten Formulierungen zum Einsatz zu bringen, wie z.B. Locron P (Clariant).

Vorteilhaft könnte auch die Verwendung von sog. AT-Salz Suspensionen sein, bei denen pulverförmig vorliegende Aluminium- und Aluminium/Zirkonium-Salze in diversen Ölen dispergiert angeboten werden.

Desweiteren kann es aber auch von Vorteil sein, spezielle Aluminium- und Aluminium/Zirkonium-Salze zum Einsatz zu bringen, die zur Löslichkeitsverbesserung als Glykol-Komplexe angeboten werden.

Weitere vorteilhafte Antitranspirant-Wirker basieren anstelle von Aluminium bzw. Zirkonium auf anderen Metallen, wie z.B. Beryllium, Titan, Hafnium.

Dabei soll die Liste der verwendbaren Antitranspirant-Wirker aber nicht auf metallhaltige Rohstoffe begrenzt sein, sondern von Vorteil sind auch Verbindungen, die Nichtmetalle wie Bor enthalten sowie solche, die dem Bereich der organischen Chemie zuzurechnen sind, wie z.B. Anticholinergika.

Vorteilhaft sind in diesem Sinne auch Polymere, die sowohl metallhaltig als auch metallfrei sein können.

Vorteilhaft können erfindungsgemäßen Zubereitungen Desodorantien zugesetzt werden. Den üblichen kosmetischen Desodorantien liegen unterschiedliche Wirkprinzipien zugrunde.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.

Alle für Desodorantien gängigen Wirkstoffe können vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, llit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Emulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Liste der genannten Wirkstoffe bzw. Wirkstoffkombinationen, die in den erfindungsgemäßen Emulsionen verwendet werden können, soll selbstverständlich nicht limitierend sein.

Die Menge der Desodorantien (eine oder mehrere Verbindungen) in den Zubereitungen beträgt vorzugsweise 0,01 bis 10 Gew.-%, bevorzugt 0,05 bis 5 Gew.-%, insbesondere bevorzugt 0,1 bis 1 Gew.-%, bezogen auf das Gesamtgewicht der Zubereitung.

Es wird bevorzugt, die Matrixphase der erfindungsgemäßen Zubereitungen zu mindestens 30,0 Gew.-%, bezogen auf das Gesamtgewicht der Zubereitungen, aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

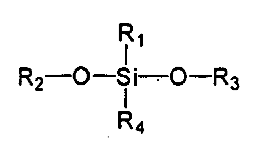

Erfindungsgemäß vorteilhaft einzusetzende lineare Silicone mit mehreren Siloxyleinheiten werden im allgemeinen durch folgendes Strukturelement charakterisiert: 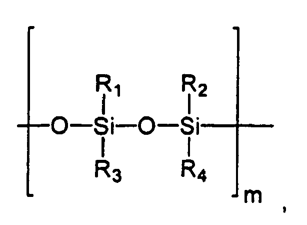 wobei die Substituenten an den Siliciumatomen R₁ bis R₄ die gleichen oder unterschiedliche Alkylreste und/oder Arylreste sein können. Die Kettenlänge m kann dabei Werte von 2 -200.000 annehmen.

Erfindungsgemäß vorteilhaft einzusetzende cyclische Silicone werden im allgemeinen durch folgendes Strukturelement charakterisiert: 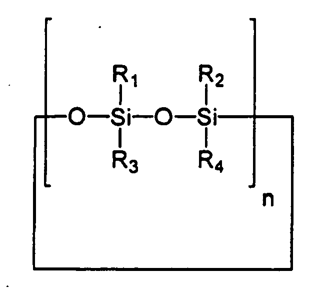 wobei die Substituenten an den Siliciumatomen R₁ bis R₄ die gleichen oder unterschiedliche Alkylreste und/oder Arylreste sein können. Die Ringgröße n kann dabei Werte von 3/2 bis 20 annehmen. Gebrochene Werte für n berücksichtigen, daß eine ungeradzahlige Anzahl von Siloxylgruppen im Cyclus vorhanden sein kann.

Vorteilhaft wird Phenyltrimethicon als Siliconöl gewählt. Auch andere Silikonöle, wie beispielsweise Dimethicon (Polydimethylsiloxan), Phenyldimethicon, Cyclomethicon (beispielsweise Hexamethylcyclotrisiloxan, Octamethylcyclotetrasiloxan, Decamethylcyclopentasiloxan, Dodecamethylcyclohexasiloxan sowie Mischungen aus diesen Komponenten), Polydimethylsiloxan, Poly(methylphenylsiloxan), Cetyldimethicon, Behenoxydimethicon sind vorteilhaft im Sinne der vorliegenden Erfindung zu verwenden. Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle von ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxanpolyalkyl-polyether-copolymere wie das Cetyl-Dimethicon-Copolyol, das (Cetyl-Dimethicon-Copolyol (und) Polyglyceryl-4-lsostearat (und) Hexyllaurat)

Es ist vorteilhaft im Sinne der vorliegenden Erfindung, die Gesamtmenge der Silikonöle auf 30 bis 85 Gew.-% zu beschränken. Erfindungsgemäß ist insbesondere eine Gesamtmenge der Silikonöle von 40 bis 80 Gew.-% und ganz besonders eine Gesamtmenge von 48 bis 72 Gew.-% - immer bezogen auf die Gesamtmenge - vorteilhaft.

Vorteilhaft kann die Matrixphase neben den Silikonölen auch einen Gehalt an anderen Ölphasenkomponenten enthalten.

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe C12-15 Alkylbenzoat, Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, Capric/Caprylic Triglycerid, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid, Octyldodekanol, Dicaprylyl Carbonat.

Es ist insbesondere vorteilhaft, wenn die Ölphase der erfindungsgemäßen Formulierungen einen Gehalt an C12-15 Alkylbenzoat aufweist. Die Gesamtmenge an C12-15 Alkylbenzoat in den fertigen Antitranspirant Zubereitungen wird vorteilhaft aus dem Bereich von 0,05 bis 2,0 Gew.-%, bevorzugt 0,5 bis 1,5 Gew.-% gewählt, bezogen auf das Gesamtgewicht der Zubereitungen.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine die nicht als Verdicker C zu verstehen sind, hydrogeniertes Polyisobuten und Isohexadekan.

Die Gesamtmenge an unpolaren Ölen, insbesondere Isohexadekan, in den fertigen Antitranspirant Zubereitungen wird vorteilhaft aus dem Bereich von 1,0 bis 10,0 Gew.-%, bevorzugt von 2,5 bis 7,5 Gew.-%, besonders bevorzugt von 4,0 bis 6,0 Gew.-% gewählt, bezogen auf das Gesamtgewicht der Zubereitungen

Der nicht-silikonhaltige flüssige Teil der Matrixphase, bevorzugt C12-15 Alkylbenzoat und Isohexadekan, verbessert die Aufschüttelbarkeit des bei längerer Nichtbenutzung sich unvermeidbar bildenden Niederschlags der suspendierten Teilchen und kaschiert darüber hinaus hervorragend den weißen Rückstand auf der Haut, der unvermeidbar einige Zeit nach Produktauftrag sichtbar wird. Dieser Rückstand besteht fast ausschließlich aus AntitranspirantWirkstoff, also zumeist Aluminium- bzw. Aluminium/Zirkonium-Salzen, und wird vom Verbraucher als äußerst störend empfunden. Eine Kaschierung erfolgt dadurch, dass die durch Abtrocknung und/oder Resorption (Aufnahme über die Haut) der flüssigen Zubereitungskomponenten zurückbleibenden Feststoffe, von diesen schwerflüchtigen und/oder schwerresorbierbaren Komponenten umhüllt werden und sich keine sichtbaren Kristalle und/oder Feststoffagglomerate bilden.

Besonders vorteilhaft sind erfindungsgemäß ferner Mischungen aus Cyclomethicon, Isohexadekan und C12-15 Alkybenzoat.

Als Packmittel für erfindungsgemäße Zubereitungen eignen sich alle Auftragsvorrichtungen für Suspensionen und/oder viskose Flüssigkeiten und/oder Gele, insbesondere Deoroller oder Auftragsvorrichtungen, bei denen getränkte Strukturkörper, insbesondere Schwämme oder Gewebe, eine Applikation der Zubereitung auf die Haut ermöglichen.

Alle Bestandteile - bis auf Wasser und Duftstoffe - der erfindungsgemäßen Suspensionen sind schwerflüchtig genug, d.h. sie weisen im reinen Zustand einen geringen Dampfdruck bei 25 °C auf, um ein Eintrocknen sowie Kristallbildung zu unterbinden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, organische Lösungsmittel oder Silikonderivate sowie Moisturizer.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispielrezepturen

### allgemeine Herstellanweisung:

Die Herstellung der erfindungsgemäßen Antitranspirant-Zubereitungen erfolgt vorteilhaft dergestalt, dass man Phase A homogenisiert und nach Zugabe von Phase B weiter homogenisiert und zum Schluss Phase C, ebenfalls unter Homogenisierung, einarbeitet.

Die Ansatzherstellung erfolgt bei Raumtemperatur ohne Erhitzen bzw. Kühlen des Ansatzes.

### Beispiel 1:

| **Phase** | **Rohstoffbezeichnung** | **Menge [%]** |
|---|---|---|
| A | Dodecamethylcyclohexasiloxan | 53,00 |
| | Polyisobuten | 0,50 |
| | Isohexadecan | 10,00 |
| | C12-15 Alkylbenzoat | 2,00 |
| | Parfüm | 1,00 |
| B | Quaternium-90 Bentonit | 2,50 |
| | Wasser | 0,50 |
| | Propylenglycolcarbonat | 0,50 |
| C | Aluminiumchlorhydroxid | 30,00 |
| | Summe | 100,00 |

### Beispiel 2:

| **Phase** | **Rohstoffbezeichnung** | **Menge [%]** |
|---|---|---|
| A | Decamethylcyclopentasiloxan und Dodecamethylcyclohexasiloxan | 63,50 |
| | Polyisobuten | 2,00 |
| | Isohexadecan | 8,00 |
| | C12-15 Alkylbenzoat | 1,50 |
| | Parfüm | 1,00 |
| B | Quaternium-90 Bentonit | 2,00 |
| | Wasser | 1,50 |
| | Propylenglycolcarbonat | 0,50 |
| C | Aluminiumchlorhydroxid | 20,00 |
| | Summe | 100,00 |

### Beispiel 3:

| **Phase** | **Rohstoffbezeichnung** | **Menge [%]** |
|---|---|---|
| A | Octamethylcyclotetrasiloxan und Decamethylcyclopentasiloxan | 57,50 |
| | Polyisobuten | 1,25 |
| | Mineralöl | 5,00 |
| | C12-15 Alkylbenzoat | 1,00 |
| | Parfüm | 1,00 |
| B | Quaternium-90 Bentonit | 2,50 |
| | Wasser | 1,00 |
| | Propylenglycolcarbonat | 0,75 |
| C | Aluminiumchlorhydroxid | 30,00 |
| | Summe | 100,00 |

### Beispiel 4:

| **Phase** | **Rohstoffbezeichnung** | **Menge [%]** |
|---|---|---|
| A | Decamethylcyclopentasiloxan und Dodecamethylcyclohexasiloxan | 68,00 |
| | Polyisobuten | 1,00 |
| | Isohexadecan | 5,00 |
| | C12-15 Alkylbenzoat | 1,00 |
| | Parfüm | 1,00 |
| B | Quaternium-90 Bentonit | 2,40 |
| | Wasser | 1,00 |
| | Propylenglycolcarbonat | 0,60 |
| C | Aluminiumchlorhydroxid | 20,00 |
| | Summe | 100,00 |

### Beispiel 5:

| **Phase** | **Rohstoffbezeichnung** | **Menge [%]** |
|---|---|---|
| A | Decamethylcyclopentasiloxan und Dodecamethylcyclohexasiloxan | 75,90 |
| | Polyisobuten | 0,50 |
| | Isohexadecan | 7,00 |
| | C12-15 Alkylbenzoat | 1,00 |
| | Parfüm | 1,00 |
| B | Quaternium-90 Bentonit | 2,80 |
| | Wasser | 1,00 |
| | Propylenglycolcarbonat | 0,80 |
| C | Aluminiumchlorhydroxid | 10,00 |
| | Summe | 100,00 |

## Patentansprüche

1. Antitranspirantzubereitung, enthaltend
ein Verdickersystem, enthaltend
- einen oder mehrere tonmineralische Verdicker A,
- ein oder mehrere Polyolefine als Verdicker B, gewählt aus der Gruppe Polyisobuten und dessen Copolymere,
- einen oder mehrere nicht-wässrige polare Aktivatoren C, gewählt wird aus der Gruppe Propylenglycolcarbonat, Ethanol, Parfümöle und
- Wasser, welches nicht in den Rohstoffen standardmässig enthalten ist
- einen oder mehrere Antitranspirantwirkstoffe und
- eine Matrixphase,
wobei die Matrixphase mindestens ein Silikonöl enthält und das Gewichtsverhältnis von tonmineralischen Verdickern A zu nicht-wässrigen polaren Aktivatoren C aus dem Bereich von 3: 1 bis 5 : 1 gewählt wird.

2. Antitranspirantzubereitung nach Anspruch 1, **dadurch gekennzeichnet, dass** die Antitranspirantwirkstoffe saure Salze sind.

3. Antitranspirantzubereitung nach einem oder mehreren der vorhergehenden Ansprüche, **dadurch gekennzeichnet, dass** der tonmineralische Verdicker A Quaternium-90 Bentonit ist.

4. Antitranspirantzubereitung nach einem oder mehreren der vorhergehenden Ansprüche, **dadurch gekennzeichnet, dass** die sauren Salze eine oder mehrere saure Aluminium- und/oder Aluminium/Zirkoniumsalze sind und die Gesamtmenge an dem oder den sauren Aluminium- und/oder Aluminium/Zirkoniumsalzen von 5 bis 40 Gew.-%, insbesondere 10 bis 25 Gew.-%, immer bezogen auf das Gesamtgewicht der Zubereitungen, gewählt wird.

5. Antitranspirantzubereitung nach einem oder mehreren der vorhergehenden Ansprüche, **dadurch gekennzeichnet, dass** die Gesamtmenge an tonmineralischen Verdickern A in der fertigen Zubereitung aus dem Bereich von 0,05 bis 5,0 Gew.-%, bevorzugt von 0,5 bis 3,5 Gew.-%, besonders bevorzugt von 1,0 bis 3,0 Gew.-% gewählt wird, immer bezogen auf das Gesamtgewicht der Zubereitungen.

6. Antitranspirantzubereitung nach einem oder mehreren der vorhergehenden Ansprüche, **dadurch gekennzeichnet, dass** die Matrixphase ein Silikonöl, insbesondere Octamethylcyclotetrasiloxan und/oder Decamethylcyclopentasiloxan und/oder Dodecamethylcyclohexasiloxan oder eine Mischung dieser Silikonöle, ist.

7. Antitranspirantzubereitung nach einem oder mehreren der vorhergehenden Ansprüche, **dadurch gekennzeichnet, dass** die Matrixphase eine Gesamtmenge an der fertigen Zubereitungen aus dem Bereich von 30 bis 85 Gew.-%, bevorzugt von 40 bis 80 Gew.-%, besonders bevorzugt von 48 bis 72 Gew.-% gewählt wird, bezogen auf das Gesamtgewicht der Zubereitungen.

8. Antitranspirantzubereitung nach einem oder mehreren der vorhergehenden Ansprüche, **dadurch gekennzeichnet, dass** die Gesamtmenge an Verdicker B in den fertigen Zubereitungen aus dem Bereich von 0,05 bis 2,0 Gew.-%, bevorzugt von 0,5 bis 1,5 Gew.-%, besonders bevorzugt wie 1,0 Gew.-% gewählt wird, bezogen auf das Gesamtgewicht der Zubereitungen.

9. Antitranspirantzubereitung nach einem oder mehreren der vorhergehenden Ansprüche, **dadurch gekennzeichnet, dass** der Verdicker B aus der Gruppe Polyisobuten und dessen Copolymere mit einem durchschnittlichen Molekulargewicht von 300 bis 5000 g/mol ist.

10. Antitranspirantzubereitung nach einem oder mehreren der vorhergehenden Ansprüche, **dadurch gekennzeichnet, dass** das Gewichtsverhältnis von tonmineralischen Verdickern A zu nicht-wässrigem polarem Aktivator C wie 4 : 1 gewählt wird.

11. Antitranspirantzubereitung nach einem oder mehreren der vorhergehenden Ansprüche, **dadurch gekennzeichnet, dass** der nicht-wässrige polare Aktivator C Propylenglycolcarbonat ist.

12. Antitranspirantzubereitung nach einem oder mehreren der vorhergehenden Ansprüche, **dadurch gekennzeichnet, dass** die Gesamtmenge an zugesetztem Wasser in den fertigen Zubereitungen aus dem Bereich von 0,01 bis 2,0 Gew.-%, bevorzugt von 0,05 bis 1,75 Gew.-%, besonders bevorzugt von 0,5 bis 1,5 Gew.-% gewählt wird, bezogen auf das Gesamtgewicht der Zubereitungen.

13. Antitranspirantzubereitung nach einem oder mehreren der vorhergehenden Ansprüche, **dadurch gekennzeichnet, dass** die Zubereitung unpolare Öle, insbesondere Isohexadecan, enthält, wobei die Gesamtmenge an unpolaren Ölen in den fertigen Zubereitungen im Bereich von 1,0 bis 10,0 Gew.-%, bevorzugt von 2,5 bis 7,5 Gew.-%, besonders bevorzugt von 4,0 bis 6,0 Gew.-% gewählt wird, bezogen auf das Gesamtgewicht der Zubereitungen.

14. Antitranspirantzubereitung nach einem oder mehreren der vorhergehenden Ansprüche, **dadurch gekennzeichnet, dass** die Zubereitungen C12-15 Alkylbenzoat enthalten, wobei die Gesamtmenge an C12-15 Alkylbenzoat in den fertigen Zubereitungen im Bereich von 0,05 bis 2,0 Gew.-%, bevorzugt von 0,5 bis 1,5 Gew.-% gewählt wird, bezogen auf das Gesamtgewicht der Zubereitungen.

15. Antitranspirantprodukt, enthaltend eine Antitranspirantzubereitung nach einem oder mehreren der vorhergehenden Ansprüche und ein Packmittel, **dadurch gekennzeichnet, dass** das Packmittel eine Auftragsvorrichtung für Suspensionen und/oder viskose Flüssigkeiten und/oder Gele ist, insbesondere ein Deoroller ist.

16. Verfahren zur Herstellung einer Antitranspirantzubereitung nach mindestens einem der Ansprüche 1 bis 15, bei dem in einem ersten Schritt eine Phase A enthaltend
- eine Matrixphase, insbesondere Silikonöl,
- mindestens einen polyolefinischen Verdicker B und
- gegebenenfalls weiteren kosmetischen Hilfs und/oder Zusatzstoffen, die nicht der Phase B oder Phase C zuzuordnen sind, homogenisiert wird
und in einem zweiten Schritt eine homogenisierte Phase B, enthaltend
- mindestens einen tonmineralischen Verdicker A,
- mindestens einen nicht-wässrigen Aktivator C und
- Wasser,
mit der Phase A vermengt und homogenisiert wird und in einem dritten Schritt mindestens ein Antitranspirantwirkstoff in die homogene Mischung der Phasen A und B suspendiert wird.

## Claims

1. Antiperspirant preparation comprising
a thickener system comprising
- one or more clay mineral thickeners A,
- one or more polyolefins as thickener B chosen from the group consisting of polyisobutene and copolymers thereof,
- one or more nonaqueous polar activators C chosen from the group consisting of propylene glycol carbonate, ethanol, perfume oils and
- water which is not present in the raw materials as a standard feature
- one or more antiperspirant active ingredients and
- a matrix phase,
where the matrix phase comprises at least one silicone oil and the weight ratio of clay mineral thickeners A to nonaqueous polar activators C is chosen from the range from 3:1 to 5:1.

2. Antiperspirant preparation according to Claim 1, **characterized in that** the antiperspirant active ingredients are acidic salts.

3. Antiperspirant preparation according to one or more of the preceding claims, **characterized in that** the clay mineral thickener A is quaternium-90 bentonite.

4. Antiperspirant preparation according to one or more of the preceding claims, **characterized in that** the acidic salts are one or more acidic aluminium and/or aluminium/zirconium salts, and the total amount of the aluminium and/or aluminium/zirconium salt(s) chosen is from 5 to 40% by weight, in particular 10 to 25% by weight, always based on the total weight of the preparations.

5. Antiperspirant preparation according to one or more of the preceding claims, **characterized in that** the total amount of clay mineral thickeners A in the finished preparation is chosen from the range from 0.05 to 5.0% by weight, preferably from 0.5 to 3.5% by weight, particularly preferably from 1.0 to 3.0% by weight, always based on the total weight of the preparations.

6. Antiperspirant preparation according to one or more of the preceding claims, **characterized in that** the matrix phase is a silicone oil, in particular octamethylcyclotetrasiloxane and/or decamethylcyclopentasiloxane and/or dodecamethylcyclohexasiloxane or a mixture of these silicone oils.

7. Antiperspirant preparation according to one or more of the preceding claims, **characterized in that** the matrix phase a total amount of the finished preparations is chosen from the range from 30 to 85% by weight, preferably from 40 to 80% by weight, particularly preferably from 48 to 72% by weight, based on the total weight of the preparations.

8. Antiperspirant preparation according to one or more of the preceding claims, **characterized in that** the total amount of thickener B in the finished preparations is chosen from the range from 0.05 to 2.0% by weight, preferably from 0.5 to 1.5% by weight, particularly preferably as 1.0% by weight, based on the total weight of the preparations.

9. Antiperspirant preparation according to one or more of the preceding claims, **characterized in that** the thickener B is from the group consisting of polyisobutene and copolymers thereof with an average molecular weight of from 300 to 5000 g/mol.

10. Antiperspirant preparation according to one or more of the preceding claims, **characterized in that** the weight ratio of clay mineral thickeners A to nonaqueous polar activator C is chosen as 4:1.

11. Antiperspirant preparation according to one or more of the preceding claims, **characterized in that** the nonaqueous polar activator C is propylene glycol carbonate.

12. Antiperspirant preparation according to one or more of the preceding claims, **characterized in that** the total amount of added water in the finished preparations is chosen from the range from 0.01 to 2.0% by weight, preferably from 0.05 to 1.75% by weight, particularly preferably from 0.5 to 1.5% by weight, based on the total weight of the preparations.

13. Antiperspirant preparation according to one or more of the preceding claims, **characterized in that** the preparation comprises nonpolar oils, in particular isohexadecane, where the total amount of nonpolar oils in the finished preparations is chosen in the range from 1.0 to 10.0% by weight, preferably from 2.5 to 7.5% by weight, particularly preferably from 4.0 to 6.0% by weight, based on the total weight of the preparations.

14. Antiperspirant preparation according to one or more of the preceding claims, **characterized in that** the preparations comprise C12-15 alkyl benzoate, where the total amount of C12-15 alkyl benzoate in the finished preparations is chosen in the range from 0.05 to 2.0% by weight, preferably from 0.5 to 1.5% by weight, based on the total weight of the preparations.

15. Antiperspirant product comprising an antiperspirant preparation according to one or more of the preceding claims and a packaging, **characterized in that** the packaging is an application device for suspensions and/or viscous liquids and/or gels, in particular is a deodorant roll-on.

16. Method of producing an antiperspirant preparation according to at least one of Claims 1 to 15, in which, in a first step, a phase A comprising
- a matrix phase, in particular silicone oil
- at least one polyolefinic thickener B and
- optionally further cosmetic auxiliaries and/or additives which are not assigned to phase B or phase C, is homogenized
- and, in a second step, a homogenized phase B comprising
- at least one clay mineral thickener A,
- at least one nonaqueous activator C and
- water,
is combined with phase A and homogenized and, in a third step, at least one antiperspirant active ingredient is suspended in the homogeneous mixture of phases A and B.

## Revendications

1. Préparation antisudorale contenant
un système épaississant contenant
- un ou plusieurs épaississants argileux A,
- une ou plusieurs polyoléfines en tant qu'épaississant B, choisies dans le groupe constitué par le polyisobutène et ses copolymères,
- un ou plusieurs activateurs polaires non aqueux C, choisis dans le groupe constitué par le carbonate de propylèneglycol, l'éthanol, les huiles essentielles et
- de l'eau, qui n'est pas normalement contenue dans les matières premières,
- un ou plusieurs actifs antisudoraux et
- une phase matrice,
la phase matrice contenant au moins une huile de silicone et le rapport pondéral des épaississants argileux A aux activateurs polaires non aqueux C étant dans la plage de 3:1 à 5:1.

2. Préparation antisudorale selon la revendication 1, **caractérisée en ce que** les actifs antisudoraux sont des sels acides.

3. Préparation antisudorale selon une ou plusieurs des revendications précédentes, **caractérisée en ce que** l'épaississant argileux A est la bentonite quaternium-90.

4. Préparation antisudorale selon une ou plusieurs des revendications précédentes, **caractérisée en ce que** les sels acides consistent en un ou plusieurs sels acides d'aluminium et/ou aluminium/zirconium et la quantité totale du ou des sels acides d'aluminium et/ou aluminium/zirconium est choisie dans la plage allant de 5 à 40 % en poids, en particulier de 10 à 25 % en poids, toujours par rapport au poids total des préparations.

5. Préparation antisudorale selon une ou plusieurs des revendications précédentes, **caractérisée en ce que** la quantité totale des épaississants argileux A dans la préparation finale est choisie dans la plage allant de 0,05 à 5,0 % en poids, de préférence de 0,5 à 3,5 % en poids, de façon particulièrement préférée de 1,0 à 3,0 % en poids, toujours par rapport au poids total des préparations.

6. Préparation antisudorale selon une ou plusieurs des revendications précédentes, **caractérisée en ce que** la phase matrice est une huile de silicone, en particulier l'octaméthylcyclotétrasiloxane et/ou le décaméthylcyclopentasiloxane et/ou le dodécaméthylcyclohexasiloxane ou un mélange de ces huiles de silicone.

7. Préparation antisudorale selon une ou plusieurs des revendications précédentes, **caractérisée en ce que** la phase matrice est présente dans les préparations finales en une quantité totale choisie dans la plage allant de 30 à 85 % en poids, de préférence de 40 à 80 % en poids, de façon particulièrement préférée de 48 à 72 % en poids, par rapport au poids total des préparations.

8. Préparation antisudorale selon une ou plusieurs des revendications précédentes, **caractérisée en ce que** la quantité totale d'épaississant B dans la préparation finale est choisie dans la plage allant de 0,05 à 2,0 % en poids, de préférence de 0,5 à 1,5 % en poids, de façon particulièrement préférée est de 1,0 % en poids, par rapport au poids total des préparations.

9. Préparation antisudorale selon une ou plusieurs des revendications précédentes, **caractérisée en ce que** l'épaississant B est choisi dans le groupe constitué par le polyisobutène et ses copolymères ayant une masse moléculaire moyenne de 300 à 5 000 g/mole.

10. Préparation antisudorale selon une ou plusieurs des revendications précédentes, **caractérisée en ce que** le rapport pondéral des épaississants argileux A à l'activateur polaire non aqueux C est choisi comme étant de 4:1.

11. Préparation antisudorale selon une ou plusieurs des revendications précédentes, **caractérisée en ce que** l'activateur polaire non aqueux C est le carbonate de propylèneglycol.

12. Préparation antisudorale selon une ou plusieurs des revendications précédentes, **caractérisée en ce que** la quantité totale d'eau ajoutée dans les préparations finales est choisie dans la plage allant de 0,01 à 2,0 % en poids, de préférence de 0,05 à 1,75 % en poids, de façon particulièrement préférée de 0,5 à 1,5 % en poids, par rapport au poids total des préparations.

13. Préparation antisudorale selon une ou plusieurs des revendications précédentes, **caractérisée en ce que** la préparation contient des huiles non polaires, en particulier de l'isohexadécane, la quantité totale des huiles non polaires dans les préparations finales étant choisie dans la plage allant de 1,0 à 10,0 % en poids, de préférence de 2,5 à 7,5 % en poids, de façon particulièrement préférée de 4,0 à 6,0 % en poids, par rapport au poids total des préparations.

14. Préparation antisudorale selon une ou plusieurs des revendications précédentes, **caractérisée en ce que** les préparations contiennent un benzoate d'alkyle en C₁₂-C₁₅, la quantité totale de benzoate d'alkyle en C₁₂-C₁₅ dans les préparations finales étant choisie dans la plage allant de 0,05 à 2,0 % en poids, de préférence de 0,5 à 1,5 % en poids, par rapport au poids total des préparations.

15. Produit antisudoral, contenant une préparation antisudorale selon une ou plusieurs des revendications précédentes et un moyen de conditionnement, **caractérisé en ce que** le moyen de conditionnement est un applicateur pour suspensions et/ou liquides visqueux et/ou gels, en particulier un applicateur à bille pour déodorant.

16. Procédé pour la production d'une préparation antisudorale selon au moins l'une des revendications 1 à 15, dans lequel, dans une première étape, on homogénéise une phase A contenant
- une phase matrice, en particulier une huile de silicone,
- au moins un épaississant polyoléfinique B et
- éventuellement d'autres adjuvants et/ou additifs cosmétiques qui ne doivent pas être adjoints à la phase B ni à la phase C,
- et, dans une deuxième étape on ajoute à et homogénéise avec la phase A une phase B homogénéisée, contenant
- au moins un épaississant argileux A,
- au moins un activateur non aqueux C et
- de l'eau,
et dans une troisième étape on met au moins un actif antisudoral en suspension dans le mélange homogène des phases A et B.
